# EUROPÄISCHE PATENTSCHRIFT

(11) **EP 3 814 471 B1**
(45) Veröffentlichungstag und Bekanntmachung des Hinweises auf die Patenterteilung: **05.03.2025**
(21) Anmeldenummer: 19734049.0
(22) Anmeldetag: 25.06.2019
(51) Int. Cl.: C11D 3/386

(54) **WASCH-ODER REINIGUNGSMITTEL ENTHALTEND POLYESTERASE**
DETERGENTS OR CLEANING AGENTS CONTAINING POLYESTERASE
DÉTERGENTS OU PRODUITS DE NETTOYAGE CONTENANT DE LA POLYESTERASE

(30) Priorität: 28.06.2018 DE 102018210608
(43) Veröffentlichungstag der Anmeldung: 05.05.2021
(73) Patentinhaber: Henkel AG & Co. KGaA, 40589 Düsseldorf (DE)
(72) Erfinder: DEGERING, Christian, 40699 Erkrath (DE); HAARMANN, Thomas, 64673 Zwingenberg (DE); ISLAM, Shohana, 52074 Aachen (DE); JAKOB, Felix, 41812 Erkelenz (DE); LORENZ, Patrick, 64653 Lorsch (DE); MUSSMANN, Nina, 47877 Willich (DE); SCHREITER, Martina, 64319 Pfungstadt (DE); SCHWANEBERG, Ulrich, 4728 Kelmis-Hergenrath (BE); SCHWERDTFEGER, Ruth, 64291 Darmstadt (DE); SEEFRIED, Michael, 63762 Großostheim (DE); WEI, Ren, 04451 Borsdorf (DE); WIELAND, Susanne, 41541 Zons/Dormagen (DE); ZIMMERMANN, Wolfgang, 04109 Leipzig (DE)
(86) Internationale Anmeldenummer: PCT/EP2019/066795
(87) Internationale Veröffentlichungsnummer: WO 2020/002310

(56) Entgegenhaltungen:
- WO-A1-2012/113827
- WO-A1-2013/033318
- WO-A1-2013/096653
- WO-A1-2017/129436

## Beschreibung

Die vorliegende Erfindung liegt auf dem Gebiet der Enzymtechnologie, insbesondere der Anti-Pilling Wirkung von Enzymen, wie sie zum Beispiel in Wasch- oder Reinigungsmitteln, verwendet werden. Die Erfindung betrifft ein Mittel, insbesondere Wasch- oder Reinigungsmittel, welches eine Polyesterase, wie hierin definiert, enthält. Ferner betrifft die vorliegende Erfindung ein Verfahren zur Reinigung von Textilien sowie die Verwendung des erfindungsgemäßen Mittels zur Entfernung von Anschmutzungen. Des Weiteren richtet sich die Erfindung auf die Verwendung einer Polyesterase zur Verringerung von Pilling-Effekten und Anti-Grau in einem Mittel, vorzugsweise einem Wasch- oder Reinigungsmittel.

Bei mehrfacher Wäsche weisen Textilien jeglicher Art mit der Zeit ein Pilling auf. Als Pilling bezeichnet man die Knötchen- oder Fusselbildung bei Stoffen. Diese kleinen Fussel entstehen insbesondere bei kurzfaserigen Stoffen. Bei langfaserigen und verzwirnten Fasern entsteht dagegen weniger Pilling. Allgemein entstehen diese Knötchen durch lose Fasern im Stoff oder solche die sich aus dem Gewebe gelöst haben. Synthetische Fasern neigen, durch ihre glatte Oberfläche, eher zum Pilling als Naturfasern, weil synthetische Fasern schneller aus dem Gewebe gelöst werden können als raue Naturfasern. Bei Wollstoffen "verfilzen" diese Fasern hauptsächlich durch mechanische Reibung und bilden Knötchen an der Oberfläche.

Die Hauptauswirkung von Pilling ist eine optische Beeinträchtigung. Durch die Knötchenbildung an der Oberfläche sehen Stoffe schnell gebraucht und älter aus als sie sind. Zusätzlich wirken farbige Textilien weniger leuchtend. Dagegen wird die Funktionalität des Stoffes kaum bis gar nicht beeinträchtigt. Pilling findet insbesondere an mechanisch stark beanspruchten Stellen statt, meist sind dies Schulter- und Bundbereich. Durch die fortlaufende Ausdünnung des Stoffes sind vor allem diese beanspruchten Bereiche gefährdet Löcher zu bilden oder gar zu reißen. Das unerwünschte Pilling hat zur Folge, dass entsprechend beeinträchtigte Textilien von den Verbrauchern schneller aussortiert und weggeworfen werden, als es auf Basis der Funktionalität des Textils notwendig wäre.

Des Weiteren neigen Textilien beim Waschen dazu zu vergrauen. Das liegt daran, dass im Waschprozess sowohl Schmutz als auch abgelöste Pigmente aus farbiger Kleidung freigesetzt werden. Obwohl versucht wird, diese durch verschiedene Waschmittelinhaltstoffe in der Waschflotte zu halten, kann trotzdem oft nicht verhindert werden, dass sich der Schmutz/die Pigmente auf der Kleidung ablagern und dort verbleiben. Dies ist der sogenannte Vergrauungs-Effekt. Besonders stark ist dieser bei einigen synthetischen Fasern wie z.B. Polyamid, aber auch Polyester ausgeprägt.

Eine technische Lösung, um den Pilling-Effekt zu verringern, liegt bislang nur für Baumwolltextilien vor. Hierbei werden Cellulasen im Reinigungsmittel verwendet, um den Pilling-Effekt zu verringern (DE 69632910 T3). Das heißt, dass Cellulasen im Waschmittel eingesetzt werden, um Anti-Pilling bzw. Anti-Grau Wirkung zu zeigen und so dafür zu sorgen, dass Kleidung länger wie neu wirkt. Cellulasen wirken jedoch ausschließlich auf Baumwolltextilien. Für andere Textilien, wie z.B. Polyestertextilien, gibt es keine vergleichbare Möglichkeit Pilling zu reduzieren. Daher ist es wünschenswert und es besteht eine Nachfrage nach Lösungen, die das Pilling von Textilien, insbesondere Textilien, die synthetische Fasern wie Polyester enthalten, zu reduzieren, um Kleidung so lange wie möglich neu aussehen zu lassen, d.h. die Farben sollen kräftig bleiben, die Form soll erhalten bleiben und die Oberflächen sollen glatt und unbeschädigt bleiben.

Ferner offenbart WO 2017/129436 A1 ein verbessertes Anti-Pilling auf Polyester durch Einsatz einer Cutinase. Cutinasen sind außerdem aus WO 2012/113827 A1 im Zusammenhang mit Verfahren zur Nahrungsmittelverarbeitung bekannt.

Überraschenderweise haben die Erfinder der vorliegenden Erfindung gefunden, dass die hierin beschriebene Polyesterase aus *Thermomonospora curvata* unter Waschprozess-Bedingungen aktiv ist und hier verschiedene pflegende Eigenschaften für PET-Textilien aufweist. Dies ist insoweit überraschend, als dass bisher bekannte Cutinasen und PET-Esterasen eher bei höheren Temperaturen (>= 60 °C) aktiv sind, und außerdem nur in der Lage sind PET sehr langsam abzubauen. Die vorliegend eingesetzte Polyesterase zeigt jedoch einen schnellen PET-Abbau bei 40 °C. Es wurde gefunden, dass das Enzym zum einen das Pilling auf neuen Polyestertextilien verhindert bzw. diesen Effekt in Kombination mit einer Cellulase auf Polyester/Baumwollmisch-Textilien unterstützt. Zum anderen können bereits gebildete Pills reduziert werden, d.h. es kann einen sogenannten "Renew"-Effekt bewirken. Des Weiteren verhindert die Polyesterase die Vergrauung von weißer Wäsche und das Verblassen/Vergrauen von farbiger Wäsche. Es wurde weiterhin gefunden, dass bei entsprechender Dosierung alle diese positiven Wascheigenschaften erreicht werden, ohne die Faser signifikant zu schädigen. Dadurch, dass die Textilien länger neu aussehen, werden sie länger getragen und werden weniger schnell ersetzt. Dies führt zu einer Reduktion des CO₂ Footprints, da weniger Polyester verbraucht wird.

Daher richtet sich in einem ersten Aspekt die vorliegende Erfindung auf einWasch- oder Reinigungsmittel, dadurch gekennzeichnet, dass es eine Polyesterase enthält, die mindestens 83 % Sequenzidentität mit der in SEQ ID NO:1 angegebenen Aminosäuresequenz über deren Gesamtlänge aufweist.

In einem weiteren Aspekt richtet sich die vorliegende Erfindung auf Verfahren zur Reinigung von Textilien, dadurch gekennzeichnet, dass in mindestens einem Verfahrensschritt ein erfindungsgemäßes Wasch- oder Reinigungsmittel angewendet wird. Die Textilien sind vorzugsweise Polyester-haltige Textilien oder bestehen aus Polyester.

In noch einem Aspekt richtet sich die vorliegende Erfindung ferner auf die Verwendung eines wie hierin beschriebenen Wasch- oder Reinigungsmittels, besonders bevorzugt flüssigen Waschmittels, zur Entfernung von Anschmutzungen.

Darüber hinaus beinhaltet ein weiterer Aspekt der Erfindung, die Verwendung der hierin beschriebenen Polyesterase zur Verringerung von Pilling-Effekten und/oder Steigerung der Anti-Grau-Wirkung eines Wasch- oder Reinigungsmittels, besonders bevorzugt eines Flüssigwaschmittels, wobei das Mittel die Polyesterase enthält.

In weiter bevorzugten Ausführungsformen umfasst die im erfindungsgemäßen Mittel enthaltene Polyesterase die in SEQ ID NO:1 angegebene Aminosäuresequenz oder besteht im Wesentlichen daraus oder besteht daraus. In verschiedenen Ausführungsformen der Erfindung umfasst die Erfindung auch Polyesterasen, die von der Aminosäuresequenz gemäß SEQ ID NO:1, beispielsweise mittels Mutagenese, abgeleitet sind.

In verschiedenen Ausführungsformen der Erfindung umfasst die Polyesterase eine Aminosäuresequenz, die zu der in SEQ ID NO:1 angegebenen Aminosäuresequenz über deren Gesamtlänge zu mindestens 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 %, 98,8 %, 99,0 %, 99,2 %, 99,4 % oder 99,6 % identisch ist oder besteht aus einer solchen Sequenz.

In verschiedenen weiteren Ausführungsformen ist das Mittel dadurch gekennzeichnet, dass
(a) die Polyesterase aus einer wie oben definierten Polyesterase als Ausgangsmolekül durch ein- oder mehrfache konservative Aminosäuresubstitution erhältlich ist; und/oder
(b) die Polyesterase aus einer wie oben definierten Polyesterase als Ausgangsmolekül durch Fragmentierung, Deletions-, Insertions- oder Substitutionsmutagenese erhältlich ist und eine Aminosäuresequenz umfasst, die über eine Länge von mindestens 180, 190, 200, 210, 220, 230, 240, 245, 250, 251, 252, 253, 254, 255, 256, 257, 258, 259, 260, 261 oder 262 zusammenhängenden Aminosäuren mit dem Ausgangsmolekül übereinstimmt.

Die erfindungsgemäßen Mittel enthalten die Polyesterase vorzugsweise in einer Menge von 0,00001 bis 1 Gew.-%, weiter bevorzugt in einer Menge von 0,0001 bis 0,5 Gew.-%, besonders bevorzugt in einer Menge von 0,001 bis 0,1 Gew.-%, jeweils bezogen auf das aktive Protein.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) "Basic local alignment search tool", J. Mol. Biol. 215:403-410, und Altschul et al. (1997) "Gapped BLAST and PSI-BLAST: a new generation of protein database search programs", Nucleic Acids Res. 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden beispielsweise die Clustal-Serie (vgl. z.B. Chenna et al. (2003) "Multiple sequence alignment with the Clustal series of programs", Nucleic Acid Res. 31:3497-3500), T-Coffee (vgl. z.B. Notredame et al. (2000) "T-Coffee: A novel method for multiple sequence alignments", J. Mol. Biol. 302:205-217) oder Programme, die auf diesen Programmen beziehungsweise Algorithmen basieren. Ferner möglich sind Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert.

Solch ein Vergleich erlaubt auch eine Aussage über die Ähnlichkeit der verglichenen Sequenzen zueinander. Sie wird üblicherweise in Prozent Identität, das heißt dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäureaustausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch in Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essentielle Funktionen aus. Es kann daher sinnvoll sein, Sequenzübereinstimmungen nur auf einzelne, gegebenenfalls kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

In verschiedenen Ausführungsformen umfasst die Polyesterase eine Aminosäuresequenz, die zu der in SEQ ID NO:1 angegebenen Aminosäuresequenz über deren Gesamtlänge zu mindestens 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 %, 98,8 %, 99,0 %, 99,2 %, 99,4 % oder 99,6 % homolog ist.

In einer weiteren Ausführungsform der Erfindung ist die Polyesterase dadurch gekennzeichnet, dass ihre Anti-Pilling-Leistung gegenüber derjenigen einer Polyesterase, die eine Aminosäuresequenz umfasst, die der in SEQ ID NO:1 angegebenen Aminosäuresequenzen entspricht, nicht signifikant verringert ist, d.h. mindestens 70 %, 75 %, 80 %, 85 %, 90 %, 95 % der Referenz-Anti-Pilling-leistung besitzt. Die Anti-Pilling-Leistung kann in einem Waschsystem bestimmt werden, das ein Waschmittel in einer Dosierung zwischen 4,5 und 7,0 Gramm pro Liter Waschflotte sowie die Polyesterase enthält, wobei die zu vergleichenden Polyesterasen konzentrationsgleich (bezogen auf aktives Protein) eingesetzt sind und die Anti-Pilling-Leistung wie hierin beschrieben ermittelt wird. Beispielsweise kann der Waschvorgang für 60 Minuten bei einer Temperatur von 60 °C erfolgen und das Wasser eine Wasserhärte zwischen 15,5° und 16,5° (deutsche Härte) aufweisen. Die Konzentration der Polyesterase in dem für dieses Waschsystem bestimmten Waschmittel beträgt von 0,00001 bis 1 Gew.-%, bevorzugt von 0,0001 bis 0,5 Gew.-%, besonders bevorzugt von 0,001 bis 0,1 Gew.-%, bezogen auf aktives, gereinigtes Protein.

Ein bevorzugtes flüssiges Waschmittel für ein solches Waschsystem ist wie folgt zusammengesetzt (alle Angaben in Gewichts-%): 4,4 % Alkylbenzolsulfonsäure, 5,6 % anionische Tenside, 2,4 % C₁₂-C₁₈ Na-Salze von Fettsäuren, 4,4 % nicht-ionische Tenside, 0,2 % Phosphonate, 1,4 % Zitronensäure, 0,95 % NaOH, 0,01 % Antischaum, 2 % Glycerin, 0,08 % Konservierungsstoffe, 1 % Ethanol, 1,6 % Enzymmix (Protease, Amylase, Cellulase, Mannanase), Rest demineralisiertes Wasser. Bevorzugt beträgt die Dosierung des flüssigen Waschmittels zwischen 4,5 und 6,0 Gramm pro Liter Waschflotte, beispielsweise 4,7, 4,9 oder 5,9 Gramm pro Liter Waschflotte. Bevorzugt wird gewaschen in einem pH-Wertebereich zwischen pH 8 und pH 10,5, bevorzugt zwischen pH 8 und pH 9.

Im Rahmen der Erfindung erfolgt die Bestimmung der Anti-Pilling-Leistung bei 60 °C unter Verwendung eines flüssigen Waschmittels wie vorstehend angegeben, wobei der Waschvorgang vorzugsweise für 60 Minuten erfolgt.

Die Anti-Pilling-Leistung kann anhand von visueller Abmusterung verfolgen. Eine Prüfergruppe ordnet in diesem Fall der zu untersuchenden Wäsche einen Wert einer Skala von 1-5 zu. Dabei steht der Wert = 1 für sehr stark gepillte Wäsche, während der Wert = 5 nicht gepillter Wäsche zugeordnet wird.

Durch den aktivitätsgleichen Einsatz der jeweiligen Polyesterase wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also beispielsweise die Anti-Pilling-Leistung, verglichen werden. Generell gilt, dass eine niedrige spezifische Aktivität durch Zugabe einer größeren Proteinmenge ausgeglichen werden kann.

Proteine können über die Reaktion mit einem Antiserum oder einem bestimmten Antikörper zu Gruppen immunologisch verwandter Proteine zusammengefasst werden. Die Angehörigen einer solchen Gruppe zeichnen sich dadurch aus, dass sie dieselbe, von einem Antikörper erkannte antigene Determinante aufweisen. Sie sind daher einander strukturell so ähnlich, dass sie von einem Antiserum oder bestimmten Antikörpern erkannt werden. Einen weiteren Erfindungsgegenstand bilden daher Polyesterasen, die dadurch gekennzeichnet sind, dass sie mindestens eine und zunehmend bevorzugt zwei, drei oder vier übereinstimmende antigene Determinanten mit einer in einem erfindungsgemäßen Mittel verwendeten Polyesterase aufweisen. Solche Polyesterasen sind auf Grund ihrer immunologischen Übereinstimmungen den in den erfindungsgemäßen Mitteln verwendeten Polyesterasen strukturell so ähnlich, dass auch von einer gleichartigen Funktion auszugehen ist.

Weitere in den erfindungsgemäßen Mitteln verwendete Polyesterasen können im Vergleich zu der in SEQ ID NO:1 beschriebenen Polyesterase weitere Aminosäureveränderungen, insbesondere Aminosäure-Substitutionen, -Insertionen oder -Deletionen, aufweisen. Solche Polyesterasen sind beispielsweise durch gezielte genetische Veränderung, d.h. durch Mutageneseverfahren, weiterentwickelt und für bestimmte Einsatzzwecke oder hinsichtlich spezieller Eigenschaften (beispielsweise hinsichtlich ihrer katalytischen Aktivität, Stabilität, usw.) optimiert. Ferner können Nukleinsäuren, die die verwendeten Polyesterasen kodieren, in Rekombinationsansätze eingebracht und damit zur Erzeugung völlig neuartiger Polyesterasen oder anderer Polypeptide genutzt werden.

Das Ziel ist es, in die bekannten Moleküle gezielte Mutationen wie Substitutionen, Insertionen oder Deletionen einzuführen, um beispielsweise die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann beispielsweise die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität der Polyesterase noch weiter erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Polyesterasen, zum Beispiel hinsichtlich ihrer Aktivität und/oder ihrer Anti-Pilling-Leistung, kann daher im Rahmen der Erfindung zusätzlich weiterentwickelt sein.

Ein weiterer Gegenstand der Erfindung ist daher ein Mittel enthaltend eine Polyesterase, die dadurch gekennzeichnet ist, dass sie aus einer Polyesterase wie vorstehend beschrieben als Ausgangsmolekül erhältlich ist durch ein- oder mehrfache konservative Aminosäuresubstitution. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z. B. der Austausch eines unpolaren Aminosäurerestes gegen einen anderen unpolaren Aminosäurerest. Konservative Aminosäuresubstitutionen im Rahmen der Erfindung umfassen beispielsweise: G=A=S, I=V=L=M, D=E, N=Q, K=R, Y=F, S=T, G=A=I=V=L=M=Y=F=W=P=S=T. Dabei ist die Homologie der derartig modifizierten Polyesterasen zu der Polyesterase mit der SEQ ID NO:1 vorzugsweise wie oben definiert.

Alternativ oder ergänzend ist die Polyesterase dadurch gekennzeichnet, dass sie aus einer in einem erfindungsgemäßen Mittel enthaltenen Polyesterase als Ausgangsmolekül durch Fragmentierung, Deletions-, Insertions- oder Substitutionsmutagenese erhältlich ist und eine Aminosäuresequenz umfasst, die über eine Länge von mindestens 180, 190, 200, 210, 220, 230, 240, 245, 250, 251, 252, 253, 254, 255, 256, 257, 258, 259, 260 oder 261 zusammenhängenden Aminosäuren mit dem Ausgangsmolekül übereinstimmt.

In verschiedenen Ausführungsformen weisen die derart erhältlichen Polyesterasen auch nach der Mutagenese/Substitution noch die hierin definierten Sequenzidentitäten von mindestens 83 % mit der Sequenz gemäß SEQ ID NO:1 auf.

So ist es beispielsweise möglich, an den Termini oder in den Loops des Enzyms einzelne Aminosäuren zu deletieren, ohne dass dadurch die hydrolytische Aktivität verloren oder vermindert wird. Ferner kann durch derartige Fragmentierung, Deletions-, Insertions- oder Substitutionsmutagenese beispielsweise auch die Allergenizität betreffender Enzyme gesenkt und somit insgesamt ihre Einsetzbarkeit verbessert werden. Vorteilhafterweise behalten die Enzyme auch nach der Mutagenese ihre hydrolytische Aktivität, d.h. ihre hydrolytische Aktivität entspricht mindestens derjenigen des Ausgangsenzyms, d.h. in einer bevorzugten Ausführungsform beträgt die hydrolytische Aktivität mindestens 80 %, vorzugsweise mindestens 90 % der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

In verschiedenen Ausführungsformen kann die Polyesterase zusätzlich zu der in SEQ ID NO:1 angegebenen Sequenz N- oder C-terminal eine oder mehrere weitere Aminosäuren aufweisen. In bestimmten Ausführungsformen kann es sich bei solchen N-terminalen Peptiden um die natürlicherweise vorkommenden Signalpeptide für die Polyesterase handeln oder auch um einen einzelnen Methionin-Rest. In solchen Ausführungsformen hat die Polyesterase beispielsweise die in SEQ ID NO:2 angegebene Aminosäuresequenz. Alle Ausführungsformen die oben im Kontext mit der maturen Polyesterase gemäß SEQ ID NO:1 offenbart sind, sind ebenso auf die Polyesterase mit der Sequenz gemäß SEQ ID NO:2 anwendbar.

Ein Gegenstand der Erfindung ist ein Mittel, das dadurch gekennzeichnet ist, dass es eine Polyesterase, wie hierin definiert, enthält. Das Mittel ist ein Wasch- oder Reinigungsmittel.

Alle Prozentangaben, die im Zusammenhang mit den hierin beschriebenen Zusammensetzungen/Mitteln gemacht werden, beziehen sich, sofern nicht explizit anders angegeben auf Gew.-%, jeweils bezogen auf die jeweilige Mischung/das jeweilige Mittel.

Im Rahmen der vorliegenden Erfindung stehen Fettsäuren bzw. Fettalkohole bzw. deren Derivate - soweit nicht anders angegeben - stellvertretend für verzweigte oder unverzweigte Carbonsäuren bzw. Alkohole bzw. deren Derivate mit vorzugsweise 6 bis 22 Kohlenstoffatomen. Insbesondere sind auch die beispielsweise nach der ROELENschen Oxo-Synthese erhältlichen Oxo-Alkohole bzw. deren Derivate entsprechend einsetzbar.

Wann immer im Folgenden Erdalkalimetalle als Gegenionen für einwertige Anionen genannt sind, so bedeutet das, dass das Erdalkalimetall natürlich nur in der halben - zum Ladungsausgleich ausreichenden - Stoffmenge wie das Anion vorliegt.

Zu diesem Erfindungsgegenstand zählen alle denkbaren Wasch- oder Reinigungsmittelarten, sowohl Konzentrate als auch unverdünnt anzuwendende Mittel, zum Einsatz im kommerziellen Maßstab, in der Waschmaschine oder in der Handwäsche. Dazu gehören beispielsweise Waschmittel für Textilien, Teppiche, oder Naturfasern, für die die Bezeichnung Waschmittel verwendet wird. Zu den Wasch- und Reinigungsmitteln im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch-und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, beispielsweise zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet.

Die erfindungsgemäßen Wasch- oder Reinigungsmittel, die als pulverförmige Feststoffe, in nachverdichteter Teilchenform, als homogene Lösungen, Gele oder Suspensionen vorliegen können, können neben der oben beschriebenen Polyesterase alle bekannten und in derartigen Mitteln üblichen Inhaltsstoffe enthalten, wobei bevorzugt mindestens ein weiterer Inhaltsstoff in dem Mittel vorhanden ist. Die erfindungsgemäßen Mittel können insbesondere Tenside, Builder (Gerüststoffe), Bleichmittel, insbesondere Persauerstoffverbindungen, oder Bleichaktivatoren enthalten. Ferner können sie wassermischbare organische Lösungsmittel, weitere Enzyme, Sequestrierungsmittel, Elektrolyte, pH-Regulatoren und/oder weitere Hilfsstoffe wie optische Aufheller, Vergrauungsinhibitoren, Schaumregulatoren sowie Farb- und Duftstoffe sowie Kombinationen hiervon enthalten.

Insbesondere eine Kombination des erfindungsgemäßen Mittels mit einem oder mehreren weiteren Inhaltsstoff(en) ist vorteilhaft, da ein solches Mittel in bevorzugten erfindungsgemäßen Ausgestaltungen eine verbesserte Reinigungsleistung durch sich ergebende Synergismen aufweist. Insbesondere durch die Kombination des erfindungsgemäßen Mittels mit einem Tensid und/oder einem Builder (Gerüststoff) und/oder einer Persauerstoffverbindung und/oder einem Bleichaktivator kann ein solcher Synergismus erreicht werden.

Vorteilhafte Inhaltsstoffe erfindungsgemäßer Mittel sind offenbart in der internationalen Patentanmeldung WO 2009/121725, dort beginnend auf Seite 5, vorletzter Absatz, und endend auf Seite 13 nach dem zweiten Absatz.

Es ist selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist. Alle Prozentangaben sind, sofern nicht anders angegeben, Gewichts-% bezogen auf das Gesamtgewicht der Zusammensetzung. Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

Die erfindungsgemäßen Mittel enthalten neben der Polyesterase vorzugsweise auch mindestens eine Verbindung aus der Klasse der Tenside, insbesondere ausgewählt aus anionischen und nichtionischen, aber auch kationischen, zwitterionischen oder amphoteren Tensiden.

Geeignete Tenside sind beispielsweise anionische Tenside der Formel (I)

R-SO₃⁻ Y⁺ (I).

In dieser Formel (I) steht R für einen linearen oder verzweigten unsubstituierten Alkylarylrest. Y⁺ steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen Y⁺ können ausgewählt sein aus NH₄⁺, ½ Zn²⁺, ½ Mg²⁺, ½ Ca²⁺, ½ Mn²⁺ und deren Mischungen.

"Alkylaryl", wie hierin verwendet, bezieht sich auf organische Reste, die aus einem Alkylrest und einem aromatischen Rest bestehen. Typische Beispiele für derartige Reste schließen ein, sind aber nicht beschränkt auf Alkylbenzolreste, wie Benzyl, Butylbenzolreste, Nonylbenzolreste, Decylbenzolreste, Undecylbenzolreste, Dodecylbenzolreste, Tridecylbenzolreste und ähnliche.

In verschiedenen Ausführungsformen sind derartige Tenside ausgewählt aus linearen oder verzweigten Alkylbenzolsulfonaten der Formel A-1 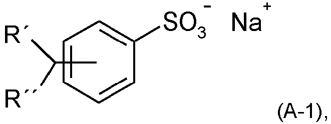 in der R' und R" zusammen 9 bis 19, vorzugsweise 11 bis 15 und insbesondere 11 bis 13 C-Atome enthalten, dargestellt. Ein ganz besonders bevorzugter Vertreter lässt sich durch die Formel A-1a beschreiben:

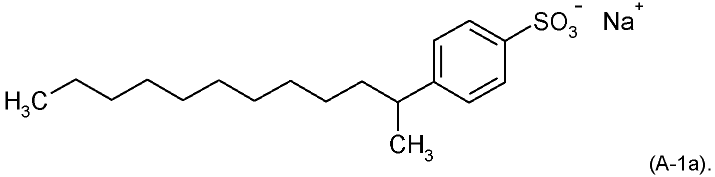

In verschiedenen Ausführungsformen handelt es sich bei der Verbindung der Formel (I) vorzugsweise um das Natriumsalz eines linearen Alkylbenzolsulfonats.

In erfindungsgemäßen Mitteln ist die mindestens eine Verbindung aus der Klasse der anionischen Tenside der Formel (I) in einer Menge von 0,001 bis 30 Gew.-%, vorzugsweise 0,001 bis 10 Gew.-%, ferner bevorzugt 2 bis 6 Gew.-%, noch bevorzugter 3 bis 5 Gew.-%, in dem Wasch- oder Reinigungsmittel enthalten, jeweils bezogen auf das Gesamtgewicht des Reinigungsmittels.

In verschiedenen Ausführungsformen enthalten die erfindungsgemäßen Mittel vorzugsweise mindestens ein anionisches Tensid der Formel

R¹-O-(AO)ₙ-SO₃⁻ X⁺ (II).

In dieser Formel (II) steht R¹ für einen linearen oder verzweigten, substituierten oder unsubstituierten Alkyl-, Aryl- oder Alkylarylrest, vorzugsweise für einen linearen, unsubstituierten Alkylrest, besonders bevorzugt für einen Fettalkoholrest. Bevorzugte Reste R¹ sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen.

AO steht für eine Ethylenoxid-(EO)- oder Propylenoxid-(PO)-Gruppierung, vorzugsweise für eine Ethylenoxidgruppierung. Der Index n steht für eine ganze Zahl von 1 bis 50, vorzugsweise von 1 bis 20 und insbesondere von 2 bis 10. Ganz besonders bevorzugt steht n für die Zahlen 2, 3, 4, 5, 6, 7 oder 8. X⁺ steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X⁺ können ausgewählt sein aus NH₄⁺, ½ Zn²⁺, ½ Mg²⁺, ½ Ca²⁺, ½ Mn²⁺ und deren Mischungen.

Zusammenfassend enthalten Mittel in verschiedenen Ausführungsformen somit mindestens ein anionisches Tensid ausgewählt aus Fettalkoholethersulfaten der Formel A-2 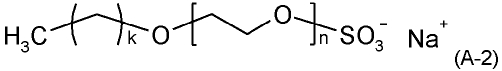 mit k = 11 bis 19, n = 2, 3, 4, 5, 6, 7 oder 8. Besonders bevorzugte Vertreter sind Na-C₁₂₋₁₄ Fettalkoholethersulfate mit 2 EO (k = 11-13, n = 2 in Formel A-2).

In verschiedenen Ausführungsformen enthält das Reinigungsmittel das mindestens ein anionisches Tensid der Formel (II) in einer Menge von 2 bis 10 Gew.-%, vorzugsweise 3 bis 8 Gew.-%, bezogen auf das Gesamtgewicht des Reinigungsmittels.

Weitere einsetzbare anionische Tenside sind die Alkylsulfate der Formel

R²-O-SO₃⁻ X⁺ (III).

In dieser Formel (III) steht R² für einen linearen oder verzweigten, substituierten oder unsubstituierten Alkylrest, vorzugsweise für einen linearen, unsubstituierten Alkylrest, besonders bevorzugt für einen Fettalkoholrest. Bevorzugte Reste R² sind ausgewählt aus Decyl-, Undecyl-, Dodecyl-, Tridecyl-, Tetradecyl-, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R² sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen. X⁺ steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X⁺ können ausgewählt sein aus NH₄⁺, ½ Zn²⁺, ½ Mg²⁺, ½ Ca²⁺, ½ Mn²⁺ und deren Mischungen.

In verschiedenen Ausführungsformen sind diese Tenside ausgewählt aus Fettalkoholsulfaten der Formel A-3 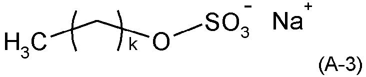 mit k = 11 bis 19. Ganz besonders bevorzugte Vertreter sind Na-C₁₂₋₁₄ Fettalkoholsulfate (k = 11-13 in Formel A-3).

In verschiedenen Ausführungsformen kann das Mittel neben den vorstehend beschriebenen Aniontensiden, insbesondere solchen der Formeln (I) bis (III), oder alternativ dazu mindestens ein anderes Tensid enthalten. Als alternative oder zusätzliche Tenside kommen insbesondere weitere anionische Tenside, nichtionische Tenside und deren Gemische, aber auch kationische, zwitterionische und amphotere Tenside in Frage.

In verschiedenen Ausführungsformen umfassen die Mittel mindestens ein nichtionisches Tensid, insbesondere mindestens ein Fettalkoholalkoxylat.

Geeignete nichtionische Tenside sind solche der Formel

R³-O-(AO)ₘ-H (IV),

in der R³ für einen linearen oder verzweigten, substituierten oder unsubstituierten Alkylrest, AO für eine Ethylenoxid-(EO)- oder Propylenoxid-(PO)-Gruppierung und m für ganze Zahlen von 1 bis 50 stehen.

In der vorstehend genannten Formel (IV) steht R³ für einen linearen oder verzweigten, substituierten oder unsubstituierten Alkylrest, vorzugsweise für einen linearen, unsubstituierten Alkylrest, besonders bevorzugt für einen Fettalkoholrest. Bevorzugte Reste R² sind ausgewählt aus Decyl-, Undecyl-, Dodecyl-, Tridecyl-, Tetradecyl, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R³ sind abgeleitet von C₁₂-C₁₈-Fettalkoholen, beispielsweise von Kokosfettalkohol, Talgfettalkohol, Lauryl-, Myristyl-, Cetyl- oder Stearylalkohol oder von C₁₀-C₂₀-Oxoalkoholen.

AO steht für eine Ethylenoxid-(EO)- oder Propylenoxid-(PO)-Gruppierung, vorzugsweise für eine Ethylenoxidgruppierung. Der Index m steht für eine ganze Zahl von 1 bis 50, vorzugsweise von 1 bis 20 und insbesondere von 2 bis 10. Ganz besonders bevorzugt steht m für die Zahlen 2, 3, 4, 5, 6, 7 oder 8.

Zusammenfassend handelt es sich bei vorzugsweise einzusetzenden Fettalkoholalkoxylaten um Verbindungen der Formel 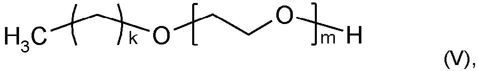 mit k = 11 bis 19, m = 2, 3, 4, 5, 6, 7 oder 8. Ganz besonders bevorzugte Vertreter sind C₁₂-₁₈ Fettalkohole mit 7 EO (k = 11-17, m = 7 in Formel (V)).

Weitere nichtionische Tenside, die im Sinne der vorliegenden Erfindung in den beschriebenen Mitteln enthalten sein können, schließen ein, sind aber nicht beschränkt auf Alkylglykoside, alkoxylierte Fettsäurealkylester, Aminoxide, Fettsäurealkanolamide, Hydroxymischether, Sorbitanfettsäurester, Polyhydroxyfettsäureamide und alkoxylierte Alkohole.

Geeignete Amphotenside sind beispielsweise Betaine der Formel (Rⁱⁱⁱ)(R^{iv})(R^{v})N⁺CH₂COO⁻, in der Rⁱⁱⁱ einen gegebenenfalls durch Heteroatome oder Heteroatomgruppen unterbrochenen Alkylrest mit 8 bis 25, vorzugsweise 10 bis 21 Kohlenstoffatomen und R^{iv} sowie R^{v} gleichartige oder verschiedene Alkylreste mit 1 bis 3 Kohlenstoffatomen bedeuten, insbesondere C₁₀-C₁₈-Alkyl-dimethylcarboxymethylbetain und C₁₁-C₁₇-Alkylamidopropyl-dimethylcarboxymethylbetain.

Geeignete Kationtenside sind u.a. die quartären Ammoniumverbindungen der Formel (R^{vi})(R^{vii})(R^{viii})(R^{ix})N⁺ X-, in der R^{vi} bis R^{ix} für vier gleich- oder verschiedenartige, insbesondere zwei lang- und zwei kurzkettige, Alkylreste und X⁻ für ein Anion, insbesondere ein Halogenidion, stehen, beispielsweise Didecyldimethylammoniumchlorid, Alkylbenzyldidecylammoniumchlorid und deren Mischungen. Weitere geeignete kationische Tenside sind die quaternären oberflächenaktiven Verbindungen, insbesondere mit einer Sulfonium-, Phosphonium-, Jodonium- oder Arsoniumgruppe, die auch als antimikrobielle Wirkstoffe bekannt sind. Durch den Einsatz von quaternären oberflächenaktiven Verbindungen mit antimikrobieller Wirkung kann das Mittel mit einer antimikrobiellen Wirkung ausgestaltet werden bzw. dessen gegebenenfalls aufgrund anderer Inhaltsstoffe bereits vorhandene antimikrobielle Wirkung verbessert werden.

In verschiedenen Ausführungsformen beträgt die Gesamtmenge der Tenside bezogen auf das Gewicht des Mittels 2 bis 30 Gew.-%, vorzugsweise 5 bis 25 Gew.-%, noch bevorzugter 10 bis 20 Gew.-%, am bevorzugtesten 14 bis 18 Gew.-%, wobei die (linearen) Alkylbenzolsulfonate höchstens in einer Menge von 0,001 bis 30 Gew.-%, vorzugsweise 0,001 bis 10 Gew.-%, ferner bevorzugt 2 bis 6 Gew.-%, noch bevorzugter 3 bis 5 Gew.-%, bezogen auf das Gewicht des Mittels vorliegen.

Erfindungsgemäße Wasch- oder Reinigungsmittel können neben der Polyesterase weitere Enzyme enthalten. Dies können hydrolytische Enzyme oder andere Enzyme in einer für die Wirksamkeit des Mittels zweckmäßigen Konzentration sein. Eine Ausführungsform der Erfindung stellen somit Mittel dar, die eines oder mehrere Enzyme umfassen. Als Enzyme bevorzugt einsetzbar sind alle Enzyme, die in dem erfindungsgemäßen Mittel eine katalytische Aktivität entfalten können, insbesondere eine Protease, Amylase, Cellulase, Hemicellulase, Mannanase, Tannase, Xylanase, Xanthanase, Xyloglucanase, β-Glucosidase, Pektinase, Carrageenase, Perhydrolase, Oxidase, Oxidoreduktase oder eine Lipase, sowie deren Gemische. Enzyme sind in dem Mittel vorteilhafterweise jeweils in einer Menge von 1 × 10⁻⁸ bis 5 Gew.-%bezogen auf aktives Protein enthalten. Zunehmend bevorzugt ist jedes Enzym in einer Menge von 1 × 10⁻⁷ bis 3 Gew.-%, von 0,00001 bis 1 Gew.-%, von 0,00005 bis 0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Besonders bevorzugt zeigen die Enzyme synergistische Reinigungsleistungen gegenüber bestimmten Anschmutzungen oder Flecken, d.h. die in der Mittelzusammensetzung enthaltenen Enzyme unterstützen sich in ihrer Reinigungsleistung gegenseitig. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Bei der/den Amylase(n) handelt es sich vorzugsweise um eine α-Amylase. Bei der Hemicellulase handelt es sich vorzugsweise um eine β-Glucanase, eine Pektinase, eine Pullulanase und/oder eine Mannanase. Bei der Cellulase handelt es sich vorzugsweise um ein Cellulase-Gemisch oder eine Einkomponenten-Cellulase, vorzugsweise bzw. überwiegend um eine Endoglucanase und/oder eine Cellobiohydrolase. Bei der Oxidoreduktase handelt es sich vorzugsweise um eine Oxidase, insbesondere eine Cholin-Oxidase, oder um eine Perhydrolase.

Die eingesetzten Proteasen sind vorzugsweise alkalische Serin-Proteasen. Sie wirken als unspezifische Endopeptidasen, das heißt, sie hydrolysieren beliebige Säureamidbindungen, die im Inneren von Peptiden oder Proteinen liegen und bewirken dadurch den Abbau proteinhaltiger Anschmutzungen auf dem Reinigungsgut. Ihr pH-Optimum liegt meist im deutlich alkalischen Bereich.

Die Proteinkonzentration kann mit Hilfe bekannter Methoden, zum Beispiel dem BCA-Verfahren (Bicinchoninsäure; 2,2'-Bichinolyl-4,4'-dicarbonsäure) oder dem Biuret-Verfahren bestimmt werden. Die Bestimmung der Aktivproteinkonzentration erfolgt diesbezüglich über eine Titration der aktiven Zentren unter Verwendung eines geeigneten irreversiblen Inhibitors (für Proteasen beispielsweise Phenylmethylsulfonylfluorid (PMSF)) und Bestimmung der Restaktivität (vgl. M. Bender et al. (1966), J. Am. Chem. Soc. 88(24):5890-5913).

In den hierin beschriebenen Reinigungsmitteln können die einzusetzenden Enzyme ferner zusammen mit Begleitstoffen, etwa aus der Fermentation, konfektioniert sein. In flüssigen Formulierungen werden die Enzyme bevorzugt als Enzymflüssigformulierung(en) eingesetzt.

Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen beispielsweise die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, beispielsweise durch Sprühtrocknung oder Extrusion der Enzymlösung zusammen mit einem vorzugsweise natürlichen Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

In verschiedenen Ausführungsformen kann das erfindungsgemäße Mittel einen oder mehrere Enzymstabilisatoren aufweisen. Daher kann das erfindungsgemäße Mittel ferner einen Enzymstabilisator beispielsweise ausgewählt aus der Gruppe bestehend aus Natriumformiat, Natriumsulfat, niederen aliphatischen Alkoholen und Borsäure sowie deren Estern und Salzen enthalten. Natürlich können auch zwei oder mehr dieser Verbindungen in Kombination eingesetzt werden. Die Salze der genannten Verbindungen können auch in Form von Hydraten, wie beispielsweise Natriumsulfat Decahydrat, eingesetzt werden.

Der Begriff "niedere aliphatische Alkohole", wie hierin verwendet, schließt Monoalkohole, Diole und höherwertige Alkohole mit bis zu 6 Kohlenstoffatomen ein. Als zu der Gruppe der niederen aliphatischen Alkohole gehörend seien in diesem Zusammenhang insbesondere Polyole, beispielsweise Glycerin, (Mono)Ethylenglykol, (Mono)Propylenglykol oder Sorbit genannt, ohne dass die Erfindung auf diese beschränkt ist.

Neben dem mindestens einen Enzymstabilisator, ausgewählt aus der obenstehenden Gruppe, kann ein erfindungsgemäßes Mittel auch mindestens einen weiteren Stabilisator enthalten. Derartige Stabilisatoren sind im Stand der Technik bekannt.

Reversible Proteaseinhibitoren schützen die in einem Wasch- oder Reinigungsmittel enthaltenen Enzyme vor proteolytischem Abbau, indem die enzymatische Tätigkeit der in dem Mittel enthaltenen Proteasen reversibel inhibiert wird. Als reversible Proteaseinhibitoren werden häufig Benzamidinhydrochlorid, Boronsäuren oder deren Salze oder Ester eingesetzt, darunter vor allem Derivate mit aromatischen Gruppen, etwa ortho-, meta- oder para-substituierte Phenylboronsäuren, insbesondere 4-Formylphenyl-Boronsäure, beziehungsweise die Salze oder Ester der genannten Verbindungen. Auch Peptidaldehyde, das heißt Oligopeptide mit reduziertem C-Terminus, insbesondere solche aus 2 bis 50 Monomeren werden zu diesem Zweck eingesetzt. Zu den peptidischen reversiblen Proteaseinhibitoren gehören unter anderem Ovomucoid und Leupeptin.

Weitere Enzymstabilisatoren sind Aminoalkohole wie Mono-, Di-, Triethanol- und -Propanolamin und deren Mischungen, aliphatische Carbonsäuren bis zu C₁₂, wie beispielsweise Bernsteinsäure, andere Dicarbonsäuren oder Salze der genannten Säuren. Auch endgruppenverschlossene Fettsäureamidalkoxylate sind für diesen Zweck geeignet. Auch manche als Builder eingesetzte organische Säuren vermögen zusätzlich ein Enzym zu stabilisieren. Auch Calcium- und/oder Magnesiumsalze werden zu diesem Zwecke eingesetzt, wie beispielsweise Calciumacetat.

Polyamid-Oligomere oder polymere Verbindungen wie Lignin, wasserlösliche Vinyl-Copolymere oder Cellulose-Ether, Acryl-Polymere und/oder Polyamide stabilisieren die Enzym-Präparation unter anderem gegenüber physikalischen Einflüssen oder pH-Wert-Schwankungen. Polyamin-N-Oxid-enthaltende Polymere wirken gleichzeitig als Enzymstabilisatoren und als Farbübertragungsinhibitoren. Andere polymere Stabilisatoren sind lineare C₈-C₁₈ Polyoxyalkylene. Auch Alkylpolyglycoside können die enzymatischen Komponenten des erfindungsgemäßen Mittels stabilisieren und vermögen vorzugsweise, diese zusätzlich in ihrer Leistung zu steigern. Vernetzte N-haltige Verbindungen erfüllen vorzugsweise eine Doppelfunktion als Soil-release-Agentien und als Enzym-Stabilisatoren. Hydrophobes, nichtionisches Polymer stabilisiert insbesondere eine gegebenenfalls enthaltene Cellulase.

Reduktionsmittel und Antioxidantien erhöhen die Stabilität der Enzyme gegenüber oxidativem Zerfall; hierfür sind beispielsweise schwefelhaltige Reduktionsmittel geläufig, etwa Natrium-Sulfit und reduzierende Zucker.

In einer Ausführungsform sind die Mittel gemäß der vorliegenden Erfindung flüssig und enthalten Wasser als Hauptlösungsmittel, d.h. es handelt sich um wässrige Mittel. Der Wassergehalt des erfindungsgemäßen wässrigen Mittels beträgt üblicherweise 15 bis 70 Gew.-%, vorzugsweise 20 bis 60 Gew.-%. In verschiedenen Ausführungsformen beträgt der Wassergehalt mehr als 5 Gew.-%, bevorzugt mehr als 15 Gew.-% und insbesondere bevorzugt mehr als 50 Gew.-%, jeweils bezogen auf die Gesamtmenge an Mittel.

Daneben können dem Mittel nichtwässrige Lösungsmittel zugesetzt werden. Geeignete nichtwässrige Lösungsmittel umfassen ein- oder mehrwertige Alkohole, Alkanolamine oder Glykolether, sofern sie im angegebenen Konzentrationsbereich mit Wasser mischbar sind. Vorzugsweise werden die Lösungsmittel ausgewählt aus Ethanol, n-Propanol, i-Propanol, Butanolen, Glykol, Propandiol, Butandiol, Methylpropandiol, Glycerin, Diglykol, Propyldiglykol, Butyldiglykol, Hexylenglykol, Ethylenglykolmethylether, Ethylenglykolethylether, Ethylenglykolpropylether, Ethylenglykolmono-n-butylether, Diethylenglykolmethylether, Diethylenglykolethylether, Propylenglykolmethylether, Propylenglykolethylether, Propylenglykolpropylether, Dipropylenglykolmonomethylether, Dipropylenglykolmonoethylether, Methoxytriglykol, Ethoxytriglykol, Butoxytriglykol, 1-Butoxyethoxy-2-propanol, 3-Methyl-3-methoxybutanol, Propylen-glykol-t-butylether, Di-n-octylether sowie Mischungen dieser Lösungsmittel.

Das eine oder die mehreren nichtwässrigen Lösungsmittel ist/sind üblicherweise in einer Menge von 0,1 bis 10 Gew.-%, vorzugsweise 1 bis 8 Gew.-% bezogen auf die Gesamtzusammensetzung enthalten.

Neben den bisher genannten Komponenten können die erfindungsgemäßen Mittel weitere Inhaltsstoffe enthalten, die die anwendungstechnischen und/oder ästhetischen Eigenschaften des Reinigungsmittels weiter verbessern. Hierzu zählen beispielsweise Additive zur Verbesserung des Ablauf- und Trocknungsverhaltens, zur Einstellung der Viskosität und/oder zur Stabilisierung, sowie weitere in Reinigungsmitteln übliche Hilfs- und Zusatzstoffe, etwa UV-Stabilisatoren, Parfüm, Perlglanzmittel, Farbstoffe, Korrosionsinhibitoren, Konservierungsmittel, Bitterstoffe, organische Salze, Desinfektionsmittel, strukturgebende Polymere, Entschäumer, verkapselte Inhaltsstoffe (z.B. verkapseltes Parfum), pH-Stellmittel sowie Hautgefühl-verbessernde oder pflegende Additive.

Ein erfindungsgemäßes Mittel, insbesondere Wasch- oder Reinigungsmittel, enthält vorzugsweise mindestens einen wasserlöslichen und/oder wasserunlöslichen, organischen und/oder anorganischen Builder (Gerüststoffe).

Zu den generell einsetzbaren Gerüststoffen zählen insbesondere die Aminocarbonsäuren und deren Salze, Zeolithe, Silikate, Carbonate, organische (Co)Builder und - wo keine ökologischen Vorurteile gegen ihren Einsatz bestehen - auch die Phosphate. Vorzugsweise sind die Mittel aber phosphatfrei.

Zu den wasserlöslichen organischen Buildersubstanzen gehören Polycarbonsäuren, insbesondere Citronensäure und Zuckersäuren, monomere und polymere Aminopolycarbonsäuren, insbesondere Methylglycindiessigsäure, Nitrilotriessigsäure und Ethylendiamintetraessigsäure sowie Polyasparaginsäure, Polyphosphonsäuren, insbesondere Aminotris(methylenphosphonsäure), Ethylendiamintetrakis(methylenphosphonsäure) und 1-Hydroxyethan-1,1-diphosphonsäure, polymere Hydroxyverbindungen wie Dextrin sowie polymere (Poly-)carbonsäuren, polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Geeignete, wenn auch weniger bevorzugte Verbindungen dieser Klasse sind Copolymere der Acrylsäure oder Methacrylsäure mit Vinylethern, wie Vinylmethylethern, Vinylester, Ethylen, Propylen und Styrol, in denen der Anteil der Säure mindestens 50 Gew.-% beträgt. Die organischen Buildersubstanzen können, insbesondere zur Herstellung flüssiger Mittel, in Form wässriger Lösungen, vorzugsweise in Form 30- bis 50-gewichtsprozentiger wässriger Lösungen eingesetzt werden. Alle genannten Säuren werden in der Regel in Form ihrer wasserlöslichen Salze, insbesondere ihre Alkalisalze, eingesetzt.

Organische Buildersubstanzen können, falls gewünscht, in Mengen bis zu 40 Gew.-%, insbesondere bis zu 25 Gew.-% und vorzugsweise von 1 bis 8 Gew.-% enthalten sein. Mengen nahe der genannten Obergrenze werden vorzugsweise in pastenförmigen oder flüssigen, insbesondere wasserhaltigen, erfindungsgemäßen Mitteln eingesetzt. Erfindungsgemäße Wäschenachbehandlungsmittel, wie z.B. Weichspüler, können gegebenenfalls auch frei von organischem Builder sein.

Als wasserlösliche anorganische Buildermaterialien kommen insbesondere Alkalisilikate und, falls keine Bedenken wegen ihres Einsatzes bestehen, auch Polyphosphate, vorzugsweise Natriumtriphosphat, in Betracht. Als wasserunlösliche, wasserdispergierbare anorganische Buildermaterialien können insbesondere kristalline oder amorphe Alkalialumosilikate, falls gewünscht, in Mengen von bis zu 50 Gew.-%, vorzugsweise nicht über 40 Gew.-% und in flüssigen Mitteln insbesondere von 1 bis 5 Gew.-%, eingesetzt werden. Unter diesen sind die kristallinen Natriumalumosilikate in Waschmittelqualität, insbesondere Zeolith A, P und gegebenenfalls X, bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm.

Geeignete Substitute beziehungsweise Teilsubstitute für das genannte Alumosilikat sind kristalline Alkalisilikate, die allein oder im Gemisch mit amorphen Silikaten vorliegen können. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 4 und y eine Zahl von 0 bis 20 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl beta- als auch delta-Natriumdisilikate (Na₂Si₂O₅ · y H₂O) bevorzugt. Auch aus amorphen Alkalisilikaten hergestellte, praktisch wasserfreie kristalline Alkalisilikate der obengenannten allgemeinen Formel, in der x eine Zahl von 1,9 bis 2,1 bedeutet, können in erfindungsgemäßen Mitteln eingesetzt werden. In einer weiteren bevorzugten Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren bevorzugten Ausführungsform erfindungsgemäßer Mittel eingesetzt. Falls als zusätzliche Buildersubstanz auch Alkalialumosilikat, insbesondere Zeolith, vorhanden ist, beträgt das Gewichtsverhältnis Alumosilikat zu Silikat, jeweils bezogen auf wasserfreie Aktivsubstanzen, vorzugsweise 1:10 bis 10:1. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1.

Buildersubstanzen sind, falls gewünscht, in den erfindungsgemäßen Mitteln vorzugsweise in Mengen bis zu 60 Gew.-%, insbesondere von 5 bis 40 Gew.-%, enthalten. Besonders bevorzugt sind in flüssigen Formulierungen wasserlösliche Builder. Erfindungsgemäße Wäschenachbehandlungsmittel, wie z.B. Weichspüler, sind vorzugsweise frei von anorganischem Builder.

Polymere Verdickungsmittel im Sinne der vorliegenden Erfindung sind die als Polyelektrolyte verdickend wirkenden Polycarboxylate, vorzugsweise Homo- und Copolymerisate der Acrylsäure, insbesondere Acrylsäure-Copolymere wie Acrylsäure-Methacrylsäure-Copolymere, und die Polysaccharide, insbesondere Heteropolysaccharide, sowie andere übliche verdickende Polymere.

Geeignete Polysaccharide bzw. Heteropolysaccharide sind die Polysaccharidgummen, beispielsweise Gummi arabicum, Agar, Alginate, Carrageene und ihre Salze, Guar, Guaran, Tragacant, Gellan, Ramsan, Dextran oder Xanthan und ihre Derivate, z.B. propoxyliertes Guar, sowie ihre Mischungen. Andere Polysaccharidverdicker, wie Stärken oder Cellulosederivate, können alternativ, vorzugsweise aber zusätzlich zu einem Polysaccharidgummi eingesetzt werden, beispielsweise Stärken verschiedensten Ursprungs und Stärkederivate, z.B. Hydroxyethylstärke, Stärkephosphatester oder Stärkeacetate, oder Carboxymethylcellulose bzw. ihr Natriumsalz, Methyl-, Ethyl-, Hydroxyethyl-, Hydroxypropyl-, Hydroxypropylmethyl- oder Hydroxyethylmethyl-Cellulose oder Celluloseacetat.

Als polymere Verdickungsmittel geeignete Acrylsäure-Polymere sind beispielsweise hochmolekulare mit einem Polyalkenylpolyether, insbesondere einem Allylether von Saccharose, Pentaerythrit oder Propylen, vernetzte Homopolymere der Acrylsäure (INCI Carbomer), die auch als Carboxyvinylpolymere bezeichnet werden.

Besonders geeignete polymere Verdickungsmittel sind aber folgende Acrylsäure-Copolymere: (i) Copolymere von zwei oder mehr Monomeren aus der Gruppe der Acrylsäure, Methacrylsäure und ihrer einfachen, vorzugsweise mit C₁₋₄-Alkanolen gebildeten, Ester (INCI Acrylates Copolymer), zu denen etwa die Copolymere von Methacrylsäure, Butylacrylat und Methylmethacrylat (CAS 25035-69-2) oder von Butylacrylat und Methylmethacrylat (CAS 25852-37-3) gehören; (ii) vernetzte hochmolekulare Acrylsäurecopolymere, zu denen etwa die mit einem Allylether der Saccharose oder des Pentaerythrits vernetzten Copolymere von C₁₀₋₃₀-Alkylacrylaten mit einem oder mehreren Monomeren aus der Gruppe der Acrylsäure, Methacrylsäure und ihrer einfachen, vorzugsweise mit C₁₋₄-Alkanolen gebildeten, Ester (INCI Acrylates/C₁₀₋₃₀ Alkyl Acrylate Crosspolymer) gehören.

Der Gehalt an polymerem Verdickungsmittel beträgt üblicherweise nicht mehr als 8 Gew.-%, vorzugsweise zwischen 0,1 und 7 Gew.-%, besonders bevorzugt zwischen 0,5 und 6 Gew.-%, insbesondere zwischen 1 und 5 Gew.-% und äußerst bevorzugt zwischen 1,5 und 4 Gew.-%, beispielsweise zwischen 2 und 2,5 Gew.-%, bezogen auf das Gesamtgewicht des Mittels.

Zur Stabilisierung des erfindungsgemäßen Mittels, insbesondere bei hohem Tensidgehalt, können ein oder mehrere Dicarbonsäuren und/oder deren Salze zugesetzt werden, insbesondere eine Zusammensetzung aus Na-Salzen der Adipin-, Bernstein- und Glutarsäure, wie sie z.B. unter dem Handelsnamen Sokalan^{®} DSC erhältlich ist. Der Einsatz erfolgt hierbei vorteilhafterweise in Mengen von 0,1 bis 8 Gew.-%, vorzugsweise 0,5 bis 7 Gew.-%, insbesondere 1,3 bis 6 Gew.-% und besonders bevorzugt 2 bis 4 Gew.-%, bezogen auf das Gesamtgewicht des Reinigungsmittels.

Kann jedoch auf deren Einsatz verzichtet werden, so ist das erfindungsgemäße Mittel vorzugsweise frei von Dicarbonsäure(salze)n.

Die erfindungsgemäßen Waschmittel können mit Referenzwaschmitteln verglichen werden, um die erhöhte Anti-Pilling-Leistung der erfindungsgemäßen Mittel festzustellen. Ein solches Waschsystem kann wie folgt zusammengesetzt sein (alle Angaben in Gewichts-%): Referenzmittel: 4,4 % Alkylbenzolsulfonsäure, 5,6 % weitere anionische Tenside, 2,4 % C₁₂-C₁₈ Na-Salze von Fettsäuren (Seifen), 4,4 % nicht-ionische Tenside, 0,2 % Phosphonate, 1,4 % Zitronensäure, 0,95 % NaOH, 0,01 % Entschäumer, 2,0 % Glycerin, 0,08 % Konservierungsstoffe, 1 % Ethanol, 1,6 % Enzymmix (Protease, Amylase, Cellulase, Mannanase), Rest demineralisiertes Wasser. Erfindungsgemäßes Mittel: 4,4 % Alkylbenzolsulfonsäure, 5,6 % weitere anionische Tenside, 2,4 % C₁₂-C₁₈ Na-Salze von Fettsäuren (Seifen), 4,4 % nicht-ionische Tenside, 0,2 % Phosphonate, 1,4 % Zitronensäure, 0,95 % NaOH, 0,01 % Entschäumer, 2,0 % Glycerin, 0,08 % Konservierungsstoffe, 1 % Ethanol, 1,6 % Enzymmix (Protease, Amylase, Cellulase, Mannanase), 0,009 % Polyesterase, Rest demineralisiertes Wasser. Bevorzugt beträgt die Dosierung des flüssigen Waschmittels zwischen 4,5 und 6,0 Gramm pro Liter Waschflotte, beispielsweise 4,7, 4,9 oder 5,9 Gramm pro Liter Waschflotte. Bevorzugt wird gewaschen in einem pH-Wertebereich zwischen pH 8 und pH 10,5, bevorzugt zwischen pH 8 und pH 9.

Die zuvor genannten Ausführungsformen der vorliegenden Erfindung umfassen alle festen, pulverförmigen, flüssigen, gelförmigen oder pastösen Darreichungsformen erfindungsgemäßer Mittel, die gegebenenfalls auch aus mehreren Phasen bestehen können sowie in komprimierter oder nicht komprimierter Form vorliegen können. Das Mittel kann als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen des Mittels zählen ferner Extrudate, Granulate, Tabletten oder Pouches. Alternativ kann das Mittel auch flüssig, gelförmig oder pastös sein, beispielsweise in Form eines nicht-wässrigen Flüssigwaschmittels oder einer nicht-wässrigen Paste oder in Form eines wässrigen Flüssigwaschmittels oder einer wasserhaltigen Paste. Weiterhin kann das Mittel als Einkomponentensystem vorliegen. Solche Mittel bestehen aus einer Phase. Alternativ kann ein Mittel auch aus mehreren Phasen bestehen. Ein solches Mittel ist demnach in mehrere Komponenten aufgeteilt (Mehrkomponentensystem).

Ein weiterer Erfindungsgegenstand ist ein Verfahren zur Reinigung von Textilien, das dadurch gekennzeichnet ist, dass in mindestens einem Verfahrensschritt ein erfindungsgemäßes Mittel angewendet wird. Die Textilien enthalten vorzugsweise Polyester oder bestehen aus diesem.

In verschieden Ausführungsformen zeichnet sich das oben beschriebene Verfahren dadurch aus, dass das erfindungsgemäße Mittel bei einer Temperatur von 0 bis 100 °C, bevorzugt 0 bis 80 °C, weiter bevorzugt 30 bis 70 °C und am meisten bevorzugt bei 40 bis 60 °C eingesetzt wird.

Hierunter fallen sowohl manuelle als auch maschinelle Verfahren, wobei maschinelle Verfahren bevorzugt sind. Verfahren zur Reinigung von Textilien zeichnen sich im Allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird. Alle denkbaren Wasch- oder Reinigungsverfahren können in wenigstens einem der Verfahrensschritte um die Anwendung eines erfindungsgemäßen Wasch-oder Reinigungsmittels bereichert werden und stellen dann Ausführungsformen der vorliegenden Erfindung dar. Alle Sachverhalte, Gegenstände und Ausführungsformen, die für erfindungsgemäße Mittel beschrieben sind, sind auch auf diesen Erfindungsgegenstand anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren gilt.

Da Enzyme natürlicherweise bereits eine katalytische Aktivität besitzen und diese auch in Medien entfalten, die sonst keine Reinigungskraft besitzen wie beispielsweise in bloßem Puffer, kann ein einzelner und/oder der einzige Schritt eines solchen Verfahrens darin bestehen, dass als einzige reinigungsaktive Komponente eine Polyesterase mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt ein erfindungsgemäßes Mittel aktiv wird. Hierunter sind Verfahren für Textilrohstoffe, Fasern oder Textilien mit synthetischen Bestandteilen bevorzugt, und ganz besonders für solche mit Polyester.

Des Weiteren erfasst die Erfindung auch die Verwendung des hierin beschriebenen Mittels, beispielsweise als Wasch- oder Reinigungsmitteln wie oben beschrieben, zur (verbesserten) Entfernung von Anschmutzungen, beispielsweise von Textilien, insbesondere Polyester-Textilien.

Schließlich bezieht sich die Erfindung auch auf die Verwendung einer Polyesterase zur Verringerung von Pilling-Effekten eines Wasch- oder Reinigungsmittels, vorzugsweise eines Waschmittels, besonders bevorzugt eines Flüssigwaschmittels, wobei das Wasch- oder Reinigungsmittel die Polyesterase enthält. Die Polyesterase ist eine Polyesterase wie sie hierin definiert ist. In verschiedenen Ausführungsformen der Verwendung ist die Polyesterase in einer Menge von 0,00001 bis 1 Gew.-%, bevorzugt in einer Menge von 0,0001 bis 0,5 Gew.-%, besonders bevorzugt in einer Menge von 0,001 bis 0,1 Gew.-%, in dem Wasch- oder Reinigungsmittel enthalten. In weiteren verschiedenen Ausführungsformen wird die Polyesterase, die eine Verringerung des Pilling-Effekts bewirkt, auf Textilien, insbesondere Textilien, die aus Polyester bestehen oder Polyester umfassen, angewendet.

Alle Sachverhalte, Gegenstände und Ausführungsformen, die für erfindungsgemäße Mittel und die Polyesterase beschrieben sind, sind auch auf die weiteren Erfindungsgegenstände anwendbar. Daher wird an dieser Stelle ausdrücklich auf die Offenbarung an entsprechender Stelle verwiesen mit dem Hinweis, dass diese Offenbarung auch für das vorstehende erfindungsgemäße Verfahren und die erfindungsgemäßen Verwendungen gilt.

### Beispiele

### Beispiel 1: Expression

Für die Expression der Polyesterase wurde ein synthetisches Gen mit einer an die *Trichoderma codon-usage* angepassten Nukleotidsequenz genutzt. Das Gen wurde mittels *Gibson-Assembly* mit verschiedenen Sekretionssignalen fusioniert und in ein Plasmid zur Amplifikation in *Escherichia coli* kloniert. Dieses Expressionsplasmid verfügt über einen starken Promotor zur Expression der entsprechenden mRNA des Polyesterasegens und über weitere Elemente, die eine Selektion von *Escherichia coli* Zellen erlauben, die das Expressionskonstrukt nach der Transformation aufgenommen haben.

Das entsprechende Konstrukt zur Transformation und anschließender Integration in das Genom von *Trichoderma reesei* wurde aus diesem Plasmid durch Restriktion mit *Not*I erhalten. Dieses Transformationsfragment beinhaltet die Elemente zur Expression des Polyesterasegens und ein Gen, welches die Selektion erfolgreich transformierter Zellen in *Trichoderma reesei* erlaubt.

Nach erfolgter Auswahl des produktivsten Expressionsstammes wurde die Polyesterase in ausreichender Menge durch Fermentation hergestellt, um für Waschanwendungstests eingesetzt werden zu können.

### Beispiel 2: Waschtest

### Verwendete Waschmittelmatrix

Dies ist eine handelsübliche Waschmittelmatrix, die für den Waschtest verwendet wurde:

| **Chemischer Name** | Gew.-% Aktivsubstanz im Rohmaterial | Gew.-% Aktivsubstanz in der Formulierung |
|---|---|---|
| Wasser demin. | 100 | Rest |
| Alkylbenzolsulfonsäure | 96 | 3-7 |
| Anionische Tenside (FAEOS) | 70 | 2-6 |
| C12-C18 Fettsäure Na-Salz | 30 | 0,3-1 |
| Nicht-ionische Tenside (FAEO) | 100 | 3-7 |
| Phosphonate | 40 | 0,1-0,8 |
| Zitronensäure | 100 | 0,1-2 |
| NaOH | 50 | 0,3-1 |
| Entschäumer | t.q. | 0,005-0,01 |
| Glycerin | 99,5 | 0,3-1 |
| Konservierungsmittel | 100 | 0,05-0,1 |
| Borsäure | 100 | 0,3-1 |
| Optischer Aufheller | 90 | 0,01-0,08 |
| Verdicker | 25 | 1-3 |
| Enzyme (außer Polyesterase) | 100 | 0,5-2 |
| Farbstoff, Parfüm | | |

| | | |
|---|---|---|
| Dosierung 50 mL | | |

### Waschtest zur Bestimmung der Anti-Pilling-Leistung von Enzymen

In einer handelsüblichen Waschmaschine werden 20 identische Versuche hintereinander durchgeführt. Als zu beurteilende Textilien werden verschiedene Polyester und Mischtextilien benutzt, welche einmal neu und einmal vorgepillt sind. Nach den 20 Versuchen wird die Pillreduktion der vorgepillten Gewebe und die Pillbildung der neuen Gewebe optisch beurteilt.

Die vorgepillten Gewebe werden durch 20-fach wiederholte Waschzyklen bei 40 °C in handelsüblichen Waschmaschinen hergestellt.

Nach jedem Waschzyklus wird die komplette Wäsche im Trockner getrocknet.

### Waschbedingungen:

Wasser mit 16° dH, 2,5 kg saubere Füllwäsche, 40 °C Normalprogramm, 50 ml Waschmittel wie oben beschrieben pro Maschine
Dosierung der zu untersuchenden Polyesterase: 50 mg Aktivenzym pro Waschmaschine
Probe 1: nur Waschmittel wie oben beschrieben (Vergleichsreferenz)
Probe 2: Waschmittel + 50 mg Polyesterase (SEQ ID NO:1) (erfindungsgemäß)

Ergebnis nach 20 Wäschen auf 100 % Polyestertextil:
Visuelle Abmusterung der Pills, Skala 1-5, sehr stark gepillt =1, nicht gepillt = 5
Probe 1: 1,6
Probe 2: 3,3
Eine Änderung von 0,5 Einheiten ist als signifikant anzusehen.

Die erfindungsgemäße Polyesterase verbessert das Pill-Erscheinungsbild signifikant.

## Patentansprüche

1. Wasch- oder Reinigungsmittel, **dadurch gekennzeichnet, dass** es eine Polyesterase enthält, die mindestens 83 % Sequenzidentität mit der in SEQ ID NO:1 angegebenen Aminosäuresequenz über deren Gesamtlänge aufweist.

2. Das Wasch- oder Reinigungsmittel nach Anspruch 1, **dadurch gekennzeichnet, dass** die Polyesterase eine Aminosäuresequenz umfasst, die zu der in SEQ ID NO:1 angegebenen Aminosäuresequenz über deren Gesamtlänge zu mindestens 84 %, 85 %, 86 %, 87 %, 88 %, 89 %, 90 %, 90,5 %, 91 %, 91,5 %, 92 %, 92,5 %, 93 %, 93,5 %, 94 %, 94,5 %, 95 %, 95,5 %, 96 %, 96,5 %, 97 %, 97,5 %, 98 %, 98,5 %, 98,8 %, 99,0 %, 99,2 %, 99,4 % oder 99,6 % identisch ist.

3. Das Wasch- oder Reinigungsmittel nach Anspruch 1 oder 2, **dadurch gekennzeichnet, dass**
(a) die Polyesterase aus einer Polyesterase nach Anspruch 1 oder 2 als Ausgangsmolekül durch ein- oder mehrfache konservative Aminosäuresubstitution erhältlich ist; und/oder
(b) die Polyesterase aus einer Polyesterase nach Anspruch 1 oder 2 als Ausgangsmolekül durch Fragmentierung, Deletions-, Insertions- oder Substitutionsmutagenese erhältlich ist und eine Aminosäuresequenz umfasst, die über eine Länge von mindestens 180, 190, 200, 210, 220, 230, 240, 245, 250, 251, 252, 253, 254, 255, 256, 257, 258, 259, 260, 261 oder 262 zusammenhängenden Aminosäuren mit dem Ausgangsmolekül übereinstimmt.

4. Das Wasch- oder Reinigungsmittel nach einem der Ansprüche 1 bis 3, wobei die Polyesterase in einer Menge von 0,00001 bis 1 Gew.-%, bevorzugt in einer Menge von 0,0001 bis 0,5 Gew.-%, besonders bevorzugt in einer Menge von 0,001 bis 0,1 Gew.-% in dem Wasch- oder Reinigungsmittel enthalten ist.

5. Das Wasch- oder Reinigungsmittel nach einem der Ansprüche 1 bis 4, **dadurch gekennzeichnet, dass**
(a) es mindestens einen zusätzlichen Inhaltsstoff ausgewählt aus der Gruppe bestehend aus Tensiden, Buildern (Gerüststoffe), Bleichmitteln, Bleichaktivatoren, wassermischbare organische Lösungsmittel, weitere Enzyme, Sequestrierungsmittel, Elektrolyte, pH-Regulatoren, optische Aufheller, Vergrauungsinhibitoren, Schaumregulatoren, Farb- und Duftstoffe sowie Kombinationen hiervon enthält;
und/oder
(b) es in fester oder flüssiger, bevorzugt flüssiger, Form vorliegt.

6. Verfahren zur Reinigung von Textilien, **dadurch gekennzeichnet, dass** in mindestens einem Verfahrensschritt ein Wasch- oder Reinigungsmittel nach einem der Ansprüche 1 bis 5 angewendet wird.

7. Verfahren nach Anspruch 6, **dadurch gekennzeichnet, dass** die Textilien Polyester enthalten oder daraus bestehen.

8. Verwendung eines Wasch- oder Reinigungsmittels nach einem der Ansprüche 1 bis 5 zur Entfernung von Anschmutzungen, insbesondere von Polyester-haltigen Textilien.

9. Verwendung einer Polyesterase, die mindestens 83 % Sequenzidentität mit der in SEQ ID NO:1 angegebenen Aminosäuresequenz über deren Gesamtlänge aufweist, zur Verringerung von Pilling-Effekten und/oder Steigerung der Anti-Grau-Wirkung eines Wasch-oder Reinigungsmittels, vorzugsweise eines Waschmittels, besonders bevorzugt eines Flüssigwaschmittels, wobei das Wasch- oder Reinigungsmittel die Polyesterase enthält.

10. Die Verwendung nach Anspruch 9, wobei
(a) die Polyesterase wie in Anspruch 2 oder 3 definiert ist; und/oder
(b) die Polyesterase in einer Menge wie in Anspruch 4 definiert in dem Wasch- oder Reinigungsmittel enthalten ist;
und/oder
(c) das Wasch- oder Reinigungsmittel wie in Anspruch 5 definiert ist.

## Claims

1. A washing or cleaning agent, **characterized in that** it comprises a polyesterase which has at least 83% sequence identity over its entire length with the amino acid sequence given in SEQ ID NO:1.

2. The washing or cleaning agent according to claim 1, **characterized in that** the polyesterase comprises an amino acid sequence which has a sequence identity of at least 84%, 85%, 86%, 87%, 88%, 89%, 90%, 90.5%, 91%, 91.5%, 92%, 92.5%, 93%, 93.5%, 94%, 94.5%, 95%, 95 .5%, 96%, 96.5%, 97%, 97.5%, 98%, 98.5%, 98.8%, 99%, 99.2%, 99.4% or 99.6%.

3. The washing or cleaning agent according to claim 1 or 2, **characterized in that**
(a) the polyesterase is obtainable from a polyesterase according to claim 1 or 2 as starting molecule by single or multiple conservative amino acid substitution; and/or
(b) the polyesterase is obtainable from a polyesterase according to claim 1 or 2 as starting molecule by fragmentation, deletion, insertion or substitution mutagenesis and comprises an amino acid sequence which, over a length of at least 180, 190, 200, 210, 220, 230, 240, 245, 250, 251, 252, 253, 254, 255, 256, 257, 258, 259, 260, 261 or 262 contiguous amino acids corresponds to the starting molecule.

4. The washing or cleaning agent according to any one of claims 1 to 3, wherein the polyesterase is present in the detergent or cleaning composition in an amount of from 0.00001 to 1% by weight, preferably in an amount of from 0.0001 to 0.5% by weight, particularly preferably in an amount of from 0.001 to 0.1% by weight.

5. The washing or cleaning agent according to any one of claims 1 to 4, **characterized in that**
(a) it comprises at least one additional ingredient selected from the group consisting of surfactants, builders, bleaching agents, bleach activators, water-miscible organic solvents, further enzymes, sequestering agents, electrolytes, pH regulators, optical brighteners, greying inhibitors, foam regulators, dyes and fragrances, and combinations thereof;
and/or
(b) it is in solid or liquid, preferably liquid, form.

6. A process for cleaning textiles, **characterized in that** a washing or cleaning agent according to one of Claims 1 to 5 is used in at least one process step.

7. A process according to Claim 6, **characterized in that** the textiles comprise polyester or consist thereof.

8. The use of a washing or cleaning agent according to one of claims 1 to 5 for removing stains, in particular from polyester-containing textiles.

9. Use of a polyesterase which has at least 83% sequence identity over its entire length with the amino acid sequence given in SEQ ID NO:1 to reduce pilling effects and/or to increase the anti-graying effect of a washing or cleaning agent, preferably a washing agent, particularly preferably a liquid washing agent, wherein the washing or cleaning agent comprises the polyesterase.

10. The use according to claim 9, wherein
(a) the polyesterase is as defined in claim 2 or 3; and/or
(b) the polyesterase is present in the detergent or cleaning agent in an amount as defined in claim 4;
and/or
(c) the detergent or cleaning agent is as defined in claim 5.

## Revendications

1. Agent de lavage ou de nettoyage, **caractérisé en ce qu'**il comprend une polyesterase qui présente une identité de séquence d'au moins 83 % sur toute sa longueur avec la séquence d'acides aminés indiquée dans SEQ ID NO:1.

2. Agent de lavage ou de nettoyage selon la revendication 1, **caractérisé en ce que** la polyesterase comprend une séquence d'acides aminés qui a une identité de séquence d'au moins 84%, 85%, 86%, 87%, 88%, 89%, 90%, 90,5%, 91%, 91,5%, 92%, 92,5%, 93%, 93,5%, 94%, 94,5%, 95%, 95 ,5%, 96%, 96,5%, 97%, 97,5%, 98%, 98,5%, 98,8%, 99%, 99,2%, 99,4% ou 99,6%.

3. Agent de lavage ou de nettoyage selon la revendication 1 ou 2, **caractérisé par le fait que**
(a) la polyesterase peut être obtenue à partir d'une polyesterase selon la revendication 1 ou 2 en tant que molécule de départ par substitution conservatrice simple ou multiple d'acides aminés ; et/ou
(b) la polyesterase peut être obtenue à partir d'une polyesterase selon la revendication 1 ou 2 comme molécule de départ par fragmentation, délétion, insertion ou mutagenèse de substitution et comprend une séquence d'acides aminés qui, sur une longueur d'au moins 180, 190, 200, 210, 220, 230, 240, 245, 250, 251, 252, 253, 254, 255, 256, 257, 258, 259, 260, 261 ou 262 acides aminés contigus, correspond à la molécule de départ.

4. L'agent de lavage ou de nettoyage selon l'une des revendications 1 à 3, dans lequel la polyesterase est présente dans le détergent ou la composition de nettoyage en une quantité allant de 0,00001 à 1% en poids, de préférence en une quantité allant de 0,0001 à 0,5% en poids, de préférence encore en une quantité allant de 0,001 à 0,1% en poids.

5. Agent de lavage ou de nettoyage selon l'une des revendications 1 à 4, **caractérisé par le fait que**
(a) il comprend au moins un ingrédient supplémentaire choisi dans le groupe constitué par les agents tensioactifs, les adjuvants, les agents de blanchiment, les activateurs de blanchiment, les solvants organiques miscibles dans l'eau, les enzymes supplémentaires, les agents séquestrants, les électrolytes, les régulateurs de pH, les azurants optiques, les inhibiteurs de grisaillement, les régulateurs de mousse, les colorants et les parfums, et les combinaisons de ces ingrédients ;
et/ou
(b) il se présente sous forme solide ou liquide, de préférence liquide.

6. Procédé de nettoyage de textiles, **caractérisé par le fait qu'**un agent de lavage ou de nettoyage selon l'une des revendications 1 à 5 est utilisé dans au moins une étape du procédé.

7. Procédé selon la revendication 6, **caractérisé par le fait que** les textiles comprennent du polyester ou sont constitués de polyester.

8. Utilisation d'un agent de lavage ou de nettoyage selon l'une des revendications 1 à 5 pour éliminer les taches, en particulier sur les textiles contenant du polyester.

9. Utilisation d'une polyesterase présentant une identité de séquence d'au moins 83 % sur toute sa longueur avec la séquence d'acides aminés indiquée dans SEQ ID NO:1 pour réduire les effets de boulochage et/ou pour augmenter l'effet anti-grisonnement d'un agent de lavage ou de nettoyage, de préférence d'un agent de lavage, en particulier d'un agent de lavage liquide, dans lequel l'agent de lavage ou de nettoyage comprend la polyesterase.

10. Utilisation selon la revendication 9, dans laquelle
(a) la polyesterase est telle que définie dans la revendication 2 ou 3 ; et/ou
(b) la polyesterase est présente dans le détergent ou l'agent de nettoyage dans une quantité telle que définie dans la revendication 4 ;
et/ou
(c) le détergent ou l'agent de nettoyage est tel que défini dans la revendication 5.
